# EUROPEAN PATENT APPLICATION

(11) **EP 2 392 560 A1**
(43) Date of publication of application: **07.12.2011**
(21) Application number: 10735493.8
(22) Date of filing: 27.01.2010
(51) Int. Cl.: C07C 62/32, B01D 11/02, A23K 1/14, C11B 1/10

(54) **METHOD FOR THE PREPARATION OF PRODUCTS HAVING A HIGH TRITERPENE CONTENT AND RESULTING PRODUCTS**

(30) Priority: 27.01.2009 ES 200900346; 28.04.2009 ES 200901156
(71) Applicant: Universidad de Granada, 18071 Granada (ES)
(72) Inventor: GARCÍA-GRANADOS LÓPEZ DE HIERRO, Andrés, 18071 Granada (ES); PARRA SÁNCHEZ, Andrés, 18071 Granada (ES); MARTÍNEZ RODRÍGUEZ, Antonio, 18071 Granada (ES); RIVAS SÁNCHEZ, Francisco, 18071 Granada (ES)
(74) Representative: Temino Ceniceros, Ignacio
(86) International application number: PCT/ES2010/000042
(87) International publication number: WO 2010/086480

(57) **Abstract**

The invention can be used to: stabilise raw materials from the olive tree, in particular stoned olive pomace and exhausted pomace, maintaining the triterpene content thereof; and prepare a product from these products, having a greater concentration of triterpenes.

## Description

### State of the art

Olive cultivation has a great importance in the temperate countries of the entire world. Its main use is olive oil, which in Spain currently produces more than one million Mt. The classic procedures for the milling of the olive and oil production are those called "three phase", in both continuous and discontinuous form. By these methods, in addition to oil, by-products such as olive juice (alpechin), aqueous fraction of the olive with or without addition of water, and the olive pomaces of various types, which are generally extracted for an additional use of oil, are obtained.

Today, in addition to the three-phase procedures, it is used the so-called "two phase" in which, in addition to oil, a mass is obtained, which contains the remains of the pulp and usually but not always, the olive pit, mixed with the water of vegetation, resulting in a by-product which is known under the name "alpeorujo (olive residue)".

From the olives is usually get, by the method currently in use so-called two phases, about 20% by weight of oil and 80% of a by-product called alpeorujo (olive residue) which has a water content of about 60%. The olive residue is dried in the olive-pomace extraction facilities for extraction with hexane in order to obtain the crude olive-pomace oil. After this process an olive oil waste "orujillo" with a dry matter content of about 32% of initial weight of the olives is industrially obtained.

This olive oil waste has a triterpene content of, though variable, about 1%.

The pit separation is usually performed to use as a fuel in various ways, remaining approximately 15% of initial weight of the olive as stoned olive pomace "orujo".

This stoned olive pomace has a triterpene content of between 1.5 and 2%, but with a very high content in rancidity, bitter, poor palatability and, tendency to fermentation products, besides having a very variable composition depending on the material background.

The current worldwide trend is to reduce the use of chemicals with prophylactic character allowing its use with therapeutic purposes only, so it has been necessary studying effective alternatives. One such alternative is the application of recent advances in nutrition to strengthen and maintain the animal health, by administering nutritional supplements, aim of which is "to provide pronutrients" to be included as ingredients in feed formulation.

The term "pronutrient" was defined, the first time, by Dr. Gordon Rosen, in the mid 1950's as a micro-ingredient included in the feed formulation in relatively small quantities with the aim of improving the physiology, intrinsic nutritional value and avoiding the presence of pathogens, classifying them according to their origin and function in four groups:
- Microbial pronutrients
- Antimicrobial pronutrients
- Food conditioners (flavorings, antioxidants, compactors)
- Prophylactics

This first classification admitted the bacterial, plant-derived, and mineral origin of the pronutrients.

From this first definition by Gordon Rosen, food industry and the law have changed notably, making necessary to revise the definition, classification and origin of the pronutrients, considering necessary to maintain the Dr. Gordon Rosen's definition and make a review of the classification and origin of pronutrients. Thus a pronutrient would continue being defined as a micro-ingredient included in the feed formulation in relatively small quantities with the object of improving the physiology, the intrinsic nutritional value and preventing the presence of pathogens.

Today it is accepted that the origin of pronutrient should be restricted to plant-derived and microbiological. This leads to consider only as pronutrients to complex organic molecules or mixtures thereof, able to regulate or stimulate the physiology without pharmacological effect or nutritional constituent. The vitamins and chelated minerals can be considered conceptually within this group, due to its inclusion as additives with a specific group within the European legislation allows considering them as close elements with its own characteristics.

On the other hand, the use of olive pomace and olive oil waste in livestock feeding is performed since immemorial time and is not subject to special regulations. Specifically, the use of stoned olive pomace is even authorized to animal feeding even in the variety more restrictive of organic farming.

For a review about the use of natural products in veterinary see http://www.racve.es/actividades/zootecnia/borrel pronutientes veterinaria.html.

The stoned olive pomace provides cellulosic material, sugars and, as has been seen, natural components of pronutrient character. However, isolation of components is costly and, moreover, the components isolated can be included in food categories for which the food regulation agencies require special authorization.

The main problem with the use of olive pomaces and olive oil waste "orujillo" animal feeding is the little control that the industrial product has, since its storage and racking produce alterations, and even extensive fermentations that alter the same, the rancidity of the fat moieties present in it, the large amount of bitter substances that reduce palatability and the presence of pit in varying amounts and with a grinding degree not very suitable for animal feed.

### Object of the invention

The present invention consists of establishing a low cost procedure to purify olive oil waste "orujillo" and olive pomaces "orujo", so that no tend to spontaneous fermentation and rancidity, and improve their palatability while keeping its character as a nutrient and pronutrient.

Likewise, the exhaustive implementation of the described procedure allows obtaining a raw material to achieve by extraction its high triterpene content substantially better than the original olive oil waste.

### Description of the invention

The claimed process consists of the combination of purification procedures by high-efficiency washing with water and ozone treatments in solid phase and/or dispersed phase in water. From a thoroughly refined olive oil waste it has been developed an economical process for the isolation of its triterpene components.

The proposed procedure comprises at least one of the phases 1 to 4, which will allow greater or lesser concentration of triterpenes in the final product obtained after phase 5:

### Phase 1. Stabilization and Stabilization of the Raw Material

This phase, in turn, comprises the following steps:
○ Grinding of the raw material
○ Exposure to an undercurrent of ozone-enriched air, preferentially ozone-enriched oxygen

### Phase 2. Removal of polar components

This phase comprises the following steps:
○ High efficiency extraction with water in a overpressure reactor
○ Filtration

### Phase 3. Treatment with ozone

This phase, in turn, comprises the following steps:
○ Treatment with water under overpressure and ozonation (ozone stream) simultaneously.
○ Filtering

### Phase 4. Centrifugation and Drying

○ Centrifugation of the resulting mass
○ Drying, preferably by atomization or another similar method

### Phase 5. Concentration

○ High efficiency extraction with ethyl acetate or other solvent of similar polarity
○ Evaporation of solvent
○ Cooling, crystallization, filtration and/or centrifugation.

Depending on the raw material used and the application of the final product, the process can be interrupted at any stage, resulting in intermediate products are also of interest.

This procedure can be applied to different products from the olive milling, preferably to olive oil waste, but also shows great efficiency on olive pomaces that can be obtained by centrifugation of olive residues or residues from the three-phases process, although in these cases their higher content in apolar products, since they have not been extracted with hexane, resulting in olive pomaces with a high terpene content but also with high fat and waxes content, among other apolar products. This makes that the subsequent aqueous purifications and the purifications with ozone are much less effective, persisting problems of rancidity, etc.

With this procedure are obtained, in particular, olive oil waste "orujillo" and olive pomaces modified with a very higher concentration of triterpenes that the concentration of triterpenes that are present in the original product.

With the procedure application without repeated washings of polar components concentrations in the final products of between 2% and 5% in triterpenes are obtained with a very low production cost, while repeating the process and in particular the phase of removal of polar components (and therefore making the procedure more expensive) this concentration can be increased.

Alternatively to Phase 5, it can be performed other processes which allow obtaining a very versatile "glycosylated extract".

To achieve the glycosylated extract, after obtaining a starting product using at least one of the first 4 phases, the following processes are performed on said base product:

### Phase 5-1. Obtaining glycosylated extract

○ High efficiency extraction with propylene glycol or other biologically acceptable glycolic solvent
○ Filtration under pressure, thereby obtaining the "glycosylated extract".
○ Washing with high efficiency water, thereby obtaining further an "aqueous glycosylated extract".

In addition, obtaining the "glycosylated extracts" described in phase 5-1 can be greatly improved by the extraction with propylene glycol basified previously with triethanolamine, which results in a considerable improvement in the extraction of terpenes with acidic character contained in olive oil waste "orujillo" with high terpene content:

### Improvement 2. Phase 5-2. Obtaining improved glycosylated extract

○ High efficiency extraction with propylene glycol or other biologically acceptable solvent previously doped with triethanolamine or other biologically acceptable organic base
○ Filtration under pressure, thereby obtaining further the "neutralized glycosylated extract".
○ Washing with high efficiency water, thereby obtaining a "neutralized aqueous glycosylated extract."

This terpene concentrate can be used directly in various applications, particularly in cosmetic, dermopharmaceutical preparations, and all those in which the propylene glycol is used as extracting agent, combined or not with triethanolamine or other biologically acceptable organic base, in which is compatible.

A third alternative to the phase 5 is to perform a highly effective aqueous extraction that allows exhausting the triterpene content of the raw material (purified olive pomace or purified olive oil waste).

### Improvement 3. Phase 5-3. High efficiency aqueous extraction

○ High efficiency aqueous extraction in basic mineral medium, typically sodium hydroxide, until slightly basic pH
○ Successive aqueous extractions, prior control of extracts, until the depletion of triterpenes in raw material.
○ Acidification of the eluates, preferably with sulfuric acid diluted up to weak acid pH.
○ Recovery of precipitates by filtration and/ or centrifugation.

This phase yields a terpene concentrate containing between 4% and 6% by weight of starting purified olive oil waste, containing more than 85% of maslinic acid and more than 10% of oleanolic acid.

This procedure for obtaining the whole of triterpene acids contained in olive oil waste without using organic solvents greatly reduces the industrial cost of the process.

These modified products that can be used directly as pronutrient in animal production, eliminate many of the problems having the use of these raw materials for animal feeding. Also, the resulting products can be used directly as an additive in feed manufacturing.

In particular, there have been produced different feeds with concentrations of between 1 g and 20 g of modified olive pomaces or olive oil waste per kg of feed.

### Embodiments of the Invention

The procedure may have at least one of the phases described. Here, there are described examples of particular conditions for each phase applied on olive oil waste, while the use of this raw material should be considered as an example and not with limitative character.

### Exemplary embodiment of the Phase 1. Solid phase stabilization

100 kg of stoned olive pomace and finely ground is subjected to a countercurrent treatment of ozone-enriched oxygen. This treatment can be performed with a filtered and dried air stream that is passed through the electrical arc producing ozone in the way it is usually performed. The control of ozone richness is controlled before and after its passage through the olive oil waste with the usual instrumentation, keeping until a decrease in consumption of ozone is appreciated.

This treatment achieves to stabilize and sterilize greatly the olive oil waste while its triterpene content remains unchanged (about 2%) and improves its odor and palatability. The product obtained with the application of this first phase could be stored with a greater guarantee and even used directly as pronutrient.

### Exemplary embodiment of the Phase 2. Removal of polar elements

100 kg of stoned olive pomace and finely ground preferably subjected to the process described in Phase 1 is subjected to a treatment with 100 kg of water in an overpressure reactor. Then it is filtered under pressure.

If the resulting mass is subjected to centrifugation and drying by atomization (Phase 4), about 50 kg of a new olive oil waste with terpenes at very good palatability and stability conditions and a triterpene content of about 3% are obtained.

Depending on the applications and the degree of purification, the washed and filtered mass under pressure can be washed several times in succession, although the repetition of the processes becomes more expensive by extractive process and cost of the washing water purification.

### Exemplary embodiment of the Phase 3. Treatment by ozonolysis

The mass of polar-washing olive oil waste from Phase 2 is treated with additional 100 kg of water, while it is carried out a treatment with ozone-enriched stream (ozonolysis).

The amount of ozone consumed will be controlled in order to decide the end of the process, very variable depending on the type of olive oil waste used. Once terminated the ozonolysis, it proceeds to the elimination of mostly of the aqueous dissolution by pressure filtration.

If the resulting mass is subjected to centrifugation and drying by atomization (Phase 4), there are obtained about 45 kg of stabilized olive oil waste and with a triterpene content of about 4%, although this value will be dependent on the type of stoned olive pomace used.

These examples can be performed on dry and olive pomace, being obtained the corresponding olive pomaces with a terpene content of between 3% and 5% depending on the procedure used, but less refined and less stable than the corresponding olive oil waste.

### Exemplary embodiment of the Phase 5. Obtaining a concentrate

100 kg of stabilized olive oil waste a with high terpene content, preferably obtained after application of the four first stages of the procedure, are subjected to a high efficiency extraction of 150 kg of ethyl acetate at 95 °C for 5 minutes. The extract thus obtained is evaporated to critical crystallization conditions, cooling the concentrate thus obtained.

A triterpene solid is achieved in this way which filtered under pressure, washed with water and dried leads to about 4.5 kg of concentrated triterpene, extraordinarily rich in acid maslinic, mainly, and oleanolic acid.

### Exemplary embodiment of the Phase 5-1. Obtaining an "glycosylated extract"

100 kg of stabilized olive oil waste s y with high terpene content, preferably obtained after application of the first 4 stages of the procedure, are subjected to a high efficiency extraction with 50 kg of propylene glycol at high temperature (preferably between 95 and 120 °C) during 5 minutes. It is thus achieve a "glycosylated extract" which may contain up to 15% of its own weight in triterpenes, although this will depend on the composition of starting purified olive oil waste , which is separated from olive oil waste by hot filtration under pressure. The rest of olive oil waste impregnated, with propylene glycol, is again treated with 50 kg of water and extracted under overpressure, filtering back under pressure and hot. In this case the extract obtained can be concentrated to the partial removal of water to obtain a diluted "glycosylated extract", which allows a further use of terpene material (about 1%), while allowing to clean the waste material from the propylene remains.

### Exemplary embodiment of the Phase 5-2. Obtaining a "neutralized glycosylated extract"

100 kg of stabilized olive oil waste s with high terpene content preferably obtained after application of the first 4 stages of the procedure are subjected to a high efficiency extraction of 50 kg of propylene glycol previously doped with approximately 1 kg of triethanolamine for 5 minutes at high temperature (preferably between 95 and 120 °C). It is thus achieved a "neutralized glycosylated extract" with a terpene content that may reach 20% of its own weight in triterpenes, which is separated from olive oil waste by filtration under pressure. The rest of olive oil waste impregnated, with propylene glycol, is again treated again with 50 kg of water and it is extracted under pressure, filtering back under overpressure. In this case the extract obtained can achieve to extract the remaining organic salts of terpenic acids, obtaining a diluted "neutralized aqueous glycosylated extract" that allows a further and full use of terpene material (about 2%), while allowing to clean the waste material from the remains of propylene glycol and triethanolamine. Proper use of propylene glycol doped with triethanolamine under the conditions described allows the depletion of original material in the acid terpenes.

### Exemplary embodiment of the Phase 5-3. Obtaining a concentrate with high terpene content by extraction in basic aqueous medium and recovery in acidic medium

100 kg of stabilized olive oil waste s with a high terpene content obtained preferably after application of the first 4 stages of the procedure, are subjected to a high efficiency extraction with 100 kg of water with 5 N sodium hydroxide at temperature of 110 °C for 5 minutes. The whole is filtered under hot pressure. The eluate obtained is acidified with 5N sulfuric acid solution up to pH 4, cooling this liquid phase up to room temperature. At this point some precipitates that are separated from the mother liquors by filtration under pressure appear. The precipitate obtained is washed with portions of 10 liters of water until the acidic medium removal of the precipitate is then dried. The basic extraction and precipitation process is repeated twice and it also proceeds to recover precipitates. It is thus obtained a solid concentrate from between 4 to 6 kg, depending on the quality of original refined olive oil waste , of solid material composed primarily of triterpene acids and a richness of more than 85% of maslinic acid and more than 10% of oleanolic acid.

## Claims

1. Method of preparation of products with high triterpenes content from products resulting from olive milling comprising at least one high efficiency extraction phase with water in an overpressure reactor.

2. Method according to previous claim further comprising the following stages prior to the extraction phase:
• Grinding of raw materials
• Treatment of the ground product by an ozone-enriched air stream, preferably enriched-ozone oxygen.

3. Method according to any of the preceding claims further comprising the next stage:
○ Treatment with water under overpressure and ozonolysis (ozone stream) simultaneously.

4. Method according to any of the preceding claims further comprising the following stages:
○ High efficiency extraction with ethyl acetate
○ Evaporation
○ Cooling and crystallization.

5. Method for the prevalence of products with high triterpene content comprising the following stages:
○ Grinding of raw materials
○ Exposure to an undercurrent of ozone-enriched air, or preferentially ozone-enriched oxygen
○ Removal of polar elements by high efficiency extraction with water in a overpressure reactor
○ Filtration
○ Treatment with water under overpressure and ozonation (ozone stream) simultaneously.
○ Filtering
○ Centrifugation of the resulting mass
○ Spray-drying or similar procedure
○ High efficiency extraction with ethyl acetate
○ Evaporation of solvent
○ Cooling, crystallization and filtration and/or centrifugation.

6. Method of preparation of products with high triterpene content from products resulting from olive milling comprising at least one high efficiency extraction phase with water in an overpressure reactor and further comprising the following processes:
• High efficiency extraction with propylene glycol or other biologically acceptable solvent
• Pressure filtration
• Washing with high efficiency water

7. Method according to previous claim **characterized in that** the solvent used in the latest high efficiency extraction has been previously doped with triethanolamine or other biologically acceptable organic base.

8. Method for the preparation of products with high triterpene content comprising the following stages:
• Grinding of raw materials
• Exposure to an undercurrent of ozone-enriched air, or preferably ozone-enriched oxygen
• Removal of polar elements by high efficiency extraction with water in an overpressure reactor
• Filtration
• Treatment with water under overpressure and ozonation (ozone stream) simultaneously
• Filtering or centrifugation of the resulting mass
• Spray-drying or similar procedure
• High efficiency extraction with propylene glycol or other biologically acceptable solvent
• Pressure filtration
• Washing with high efficiency water

9. Method according to previous claim **characterized in that** the solvent used in the latest high efficiency extraction has been previously doped with triethanolamine or other biologically acceptable organic base

10. Method of preparation of products with high content of triterpenes from products resulting from olive milling comprising at least one high efficiency extraction phase with water in an overpressure reactor and further comprising the following processes:
○ High efficiency aqueous extraction in basic mineral medium, typically of sodium hydroxide to slightly basic pH
○ Successive aqueous extractions until triterpenes depletion in the raw material.
○ Acidification of the eluates or, preferably with sulfuric acid diluted up to weal acid pH
○ Recovery of precipitates by filtration and/or centrifugation.

11. Method for the prevalence of products with high triterpene content comprising the following stages:
○ Grinding of raw materials
○ Exposure to an undercurrent of ozone-enriched air, or preferably ozone-enriched oxygen
○ Removal of polar elements by high efficiency extraction with water in a overpressure reactor
○ Filtration
○ Treatment with water under overpressure and ozonation (ozone stream) simultaneously
○ Filtering
○ Centrifugation of the resulting mass
○ High efficiency aqueous extraction in basic mineral medium, typically of sodium hydroxide to slightly basic pH
○ Successive aqueous extractions until triterpene depletion in the raw material.
○ Acidification of the eluates or, preferably with sulfuric acid diluted up to weal acid pH
○ Recovery of precipitates by filtration and/or centrifugation.

12. Procedures for obtaining a terpene concentrate with richness greater than 95% without the use of organic solvents from the exhausted olive pomaces with a high terpene content by extracting in basic medium of highly purified exhausted olive pomaces with a high terpene content, precipitation by acidification of the basic extract and isolation of the terpene concentrate by filtration and/or centrifugation and drying.

13. Modified exhausted olive pomace obtained by a method according to any of the preceding claims which has a triterpene content higher than 2%.

14. Modified exhausted olive pomace obtained by a method according to any of the preceding claims which has a triterpene content of between 2% and 5%.

15. Modified olive pomace obtained by a method according to any of the preceding claims which has a triterpene content higher than 2%.

16. Modified olive pomace obtained by a method according to any of the preceding claims which has a triterpene content between 2% and 5%.

17. Use of the product obtained by a method according to any of the claims as pronutrient technically improved in animal feeding.

18. Use of stabilized, rich triterpenes exhausted olive pomace obtained by a method according to claims 2 to 5 as ingredient technically improved in animal feeding.

19. Feed additived with the pronutrient according to any of claims 13 to 16 for animal production.

20. Feed for animal production according to previous claim containing concentrations of between 1 and 20 g of exhausted olive pomaces modified according to claims 2, 3, 4 and 5 kg of feed.

21. Feed for animal production according to any of claim 19 containing concentrations between 1 and 20 g of olive pomaces modified according to claim 2, 3, 4 and 5 kg of feed.

22. Glycosylated triterpene extract obtained by the method according to any of claims 6 to 9 containing a triterpene content higher than 10%.

23. Triterpene extract obtained by the method according to any of claims 10, 11 or 12 having a triterpene content with richness higher than 95%.

24. Triterpene extract obtained by the method according to any of claim 10, 11 or 12 having a maslinic acid content higher than 85% and oleanolic acid content higher than 10%.

25. Direct use of the stabilized, rich in triterpenes material obtained by the method according to claims 6 to 10 in cosmetic, dermopharmaceutical preparations and all those in which propylene glycol is used as a carrier, combined or not with triethanolamine or other biologically acceptable organic base.
